# EUROPEAN PATENT SPECIFICATION

(11) **EP 0 720 457 B1**
(45) Date of publication and mention of the grant of the patent: **09.05.2001**
(21) Application number: 94929175.1
(22) Date of filing: 12.09.1994
(51) Int. Cl.: A61F 5/00, A61F 5/01

(54) **INFLATABLE CERVICAL TRACTION/STRETCH DEVICE**
AUFBLASBARE, ZERVIKALE ZUG/STRECKVORRICHTUNG
APPAREIL DE TRACTION/D'EXTENSION CERVICALE GONFLABLE

(30) Priority: 13.09.1993 US 120602; 09.09.1994 US 303691
(43) Date of publication of application: 10.07.1996
(73) Proprietor: GLACIER CROSS, INC., Kalispell, MT 59901 (US)
(72) Inventor: CHITWOOD, Ralph, M., Whitefish, MT 59937 (US)
(74) Representative: Schuhmann, Albrecht
(86) International application number: US9410237
(87) International publication number: WO9507669

(56) References cited:
- DE-A- 3 318 938
- DE-A- 3 905 115
- US-A- 3 343 532
- US-A- 3 892 229
- US-A- 4 099 523
- US-A- 4 123 104
- US-A- 4 805 603
- US-A- 5 181 452

## Description

### BACKGROUND OF THE INVENTION

### 1. Field of the Invention.

The present invention relates to an inflatable, one-piece cervical traction/stretch device which is positioned under the neck of a user lying on a flat surface and between the shoulders and the head of the user and includes an expandable, inflatable body made of a elastically expandable material and having a shoulder portion, a bellows portion and a head portion. A hand operated bulb type air pump with a manually operated air pressure relief valve is connected to the body for manually filing the head and shoulder portion and for expanding and contracting the bellows portion thereby to stretch the neck and release stretching force on the neck.

### 2. Description of the related art.

Heretofore it has been proposed to provide a traction pillow and a inflatable cervical traction pillow. Examples of such pillows are disclosed in the following two U.S. Patents:

| U.S.Patent No. | Patentee |
|---|---|
| 5,060,661 | Howard |
| 4,832,007 | Davis, Jr. et al. |
| 4,805,603 | Cumberland |
| 4,771,493 | Park |
| 4,732,144 | Cunanan |

DE-A-39 05 115 discloses a U-shaped cervical traction or stretch device with an upper part and a lower part, whereby both parts can be extended from each other by a pumping mechanism consisting of a plurality of lifting cylinders.

### SUMMARY OF THE INVENTION

According to the present invention there is provided a cervical traction or stretch device as claimed in claim 1.

According to a preferred embodiment of the present invention there is provided a cervical traction/stretch device comprising a hollow inflatable body made of an elastically expandable material. The body includes a shoulder portion, a head portion and a bellows portion. The bellows portion, the shoulder portion and the head portion have aligned U-shaped openings therein adapted to receive and support a patient's neck. Air pump and relief structure are connected to the body, for pumping air into the body and for relieving or releasing air out of the body. The bellows portion has an un-inflated condition, a first inflated condition and a second inflated condition where the bellows portion is expanded and acts against and between the head portion and the shoulder portion.

### BRIEF DESCRIPTION OF THE DRAWINGS

FIG. 1 is a perspective view of the inflatable cervical traction/stretch device constructed according to the teachings of the present invention.

FIG. 2 is a top plan view of a body of the inflatable cervical traction/stretch device shown in FIG. 1.

FIG. 3 is an end plan view of the body of the cervical traction/stretch device shown in FIG. 2 and is taken along line 3-3 of FIG. 2.

FIG. 4 is a sectional view through the body of the cervical traction/stretch device shown in FIG. 2 and is taken along line 4-4 of FIG. 2.

FIG. 5 is a sectional view through the body of the cervical traction/stretch device shown in FIG. 2 and is taken along line 5-5 of FIG. 2.

FIG. 6 is a sectional view through the body of the cervical traction/stretch device shown in FIG. 2 and is taken along line 6-6 of FIG. 2.

FIG. 7 is a sectional view through a head portion of the body of the cervical traction/stretch device and is taken along line 7-7 of FIG. 3.

FIG. 8 is a side plan view of the cervical traction/stretch device with the neck of a patient resting therein and with the device being in a inflated but non-extended condition.

FIG. 9 is a side elevational view of a patient resting in the cervical traction/stretch device, similar to the view shown in FIG. 8, but showing the cervical traction/stretch device in an inflated and extended condition for stretching or placing traction on the patient's neck.

### DESCRIPTION OF THE PREFERRED EMBODIMENT

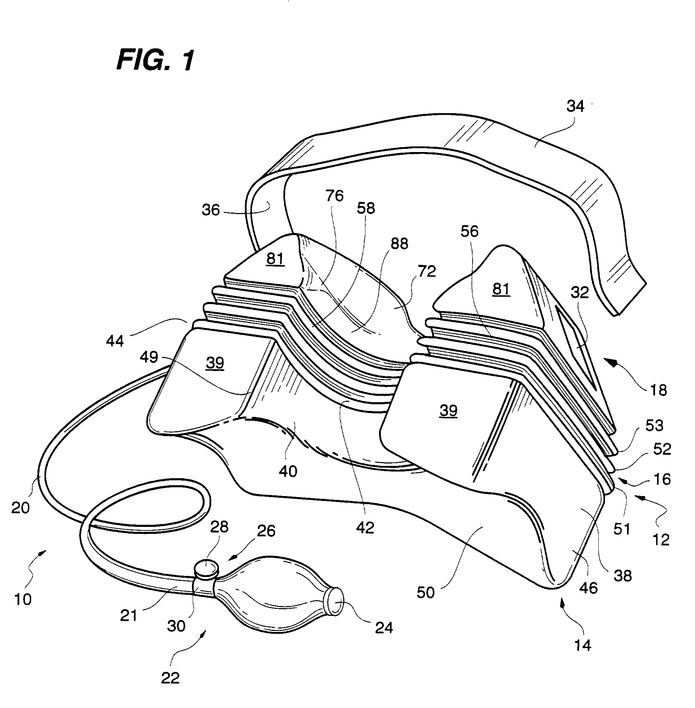

Referring now to the drawings in greater detail there is illustrated in FIG. 1 an inflatable cervical traction/stretch device 10 constructed according to the teachings of the present invention. The device 10 includes a body 12 including a shoulder portion 14, a bellows portion 16, and a head portion 18.

As shown in FIG. 1, the device is inflated in a first inflated condition from an un-inflated condition (not shown) in which the shoulder portion 14, the bellows portion 16 and the head portion 18 are completely deflated.

The shoulder portion 14, the bellows portion 16 and the head portion 18 are made of an elastically expandable material, such as rubber, and when in the first inflated condition, have sufficient rigidity or hardness to establish traction, stretching or lift surfaces thereby to enable maximum traction forces to be created.

The cervical traction/stretch device 10 further includes a tubing 20 connected to the bellows portion 16 and having, at an outer end 21 thereof, an air pump 22 in the form of compressible bulb 22 for pumping the cervical traction/stretch device 10 with air. The compressible bulb 22 has, at its outer end, a one way inlet valve 24 which allows air to be sucked into the bulb 22, but does not allow air to flow out of the bulb 22 when it is compressed.

Adjacent to the bulb 22 and mounted on the tubing 20 is a relief valve 26 which comprises a knurled thumbscrew 28 mounted in a metal collar 30 fixed to the tubing 20. When the thumbscrew 28 is rotated into the collar 30, no air can escape from the cervical traction/stretch device 10 and when the thumbscrew 28 is threaded outwardly, the valve 26 is opened to allow compressed air to escape from the cervical traction/stretch device 10 through the tubing 20 and out of the relief valve 26.

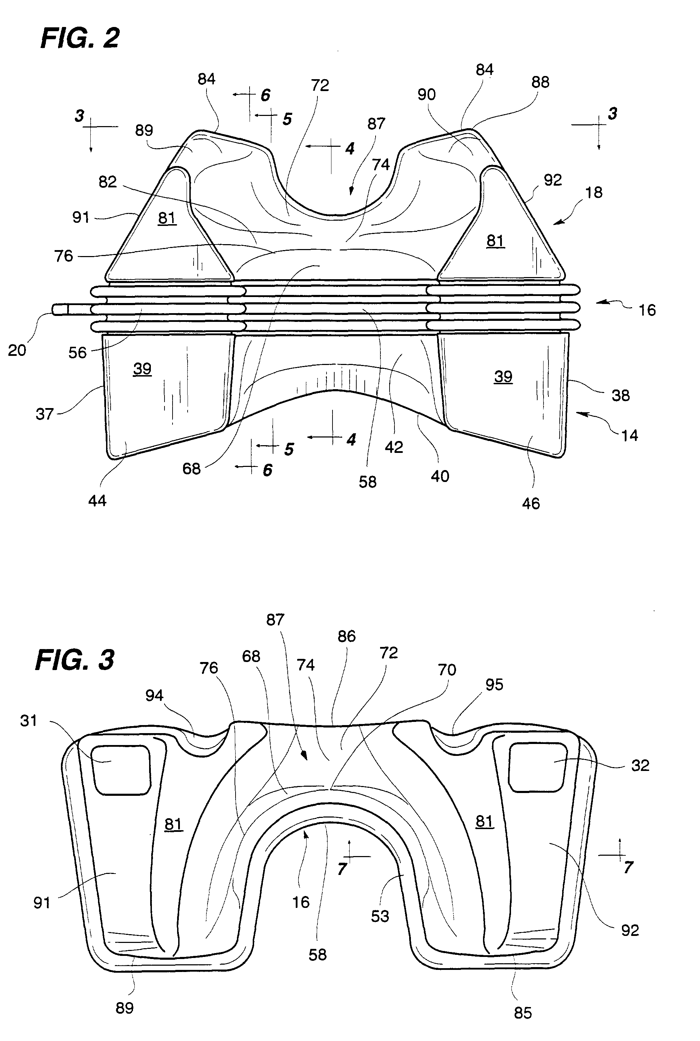

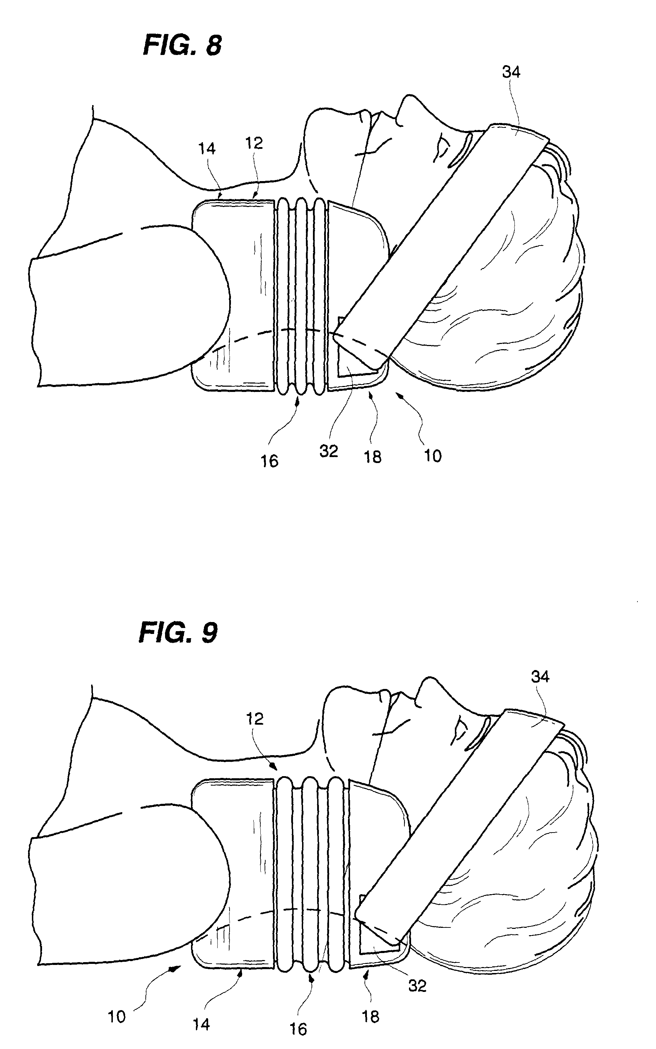

In addition to the compressible bulb 22 and the tubing 20, the cervical traction/stretch device 10 includes, on either side of the head portion 18, loop and hook type fastening structure 31, 32 (FIG. 3) of the type sold under the trademark VELCRO® and a head strap 34 which is adapted to be received over a patient's head. The strap 34 has on its inner surface 36 a fabric texture which is adapted to attach to the fastening structures 31 and 32. This is shown in FIGS. 8 and 9 where there is shown a patient's head resting in the cervical traction/stretch device 10 with the shoulder portion 14 bearing against the patient's shoulders and the patient's head being received in the head portion 18 with the head strap 34 extending over the forehead and being connected to the fastening structures 31 and 32 on either side of the head portion 18.

When inflated to a first condition, as shown in FIGS. 2-7, the shoulder portion 14 is contoured to rest on the shoulders of a patient for enabling a counter-stretch force to be created by the cervical traction/stretch device 10. In this respect, it will be noted that the shoulder portion 14, the bellows portion 16 and the hand portion 18 have various specially shaped curved surfaces for allowing the device 10 to create comfortable and therapeutic stretching to a patient's neck. These specially shaped curved surfaces are described below briefly and are described in detail in the parent application, U.S. Serial No. 08/120,602 filed on September 13, 1993 of which this application is a continuation-in-part.

The shoulder portion 14 has a bottom 36, opposite end walls 37 and 38, a contoured curved outer end surface 40 and an arcuate, semi-cylindrical or U-shaped surface 42 that extends downwardly from the top side 39 in the middle area thereof between two spaced apart block portions 44 and 46 of the shoulder portion 14.

As best shown in FIG. 1, the outer end surface 40 of the shoulder portion 14 inclines slightly inwardly towards the bellows portion 16 from each side wall 37 and 38 of the shoulder portion 14 to the middle of the shoulder portion 14 to accommodate the natural sloping of a patient's shoulders. Also from top to bottom, the contoured end surface 40 is curved inwardly and then outwardly to form arcuate surfaces 49 or 50 on the respective block portions 44 and 46 which are received over the natural contour of a patient's left and right shoulders. The extent of the U-shaped surface 42 varies from approximately 1" in the middle area of the body 12 to approximately 2" at the top side 39.

The construction of the curved outer end surface 40 of the shoulder portion 14 enables the shoulder portion 14 to fit easily over the front and center portions of the shoulders of a patient or user to enable a better and more manageable stretch force to be applied against the patient's shoulders with a minimum of slippage.

The bellows portion 16 is constructed with a plurality, e.g., three undulations 51, 52 and 53 in the illustrated embodiment and is constructed and arranged to raise and support the cervical curve of a patient's neck during inflation. Also, to provide an even force along the width of the shoulder portion 14 and most importantly, along the width of the head portion 18.

The bellows portion 16 is generally rectangular and extends substantially the full height and width of the body 12 of the device 10. The bellows portion 16 has a top side 56 and an arcuate, semi-circular or U-shaped surface 58 extending downwardly from the top side 56 generally aligned with the U-shaped surface 42 of the shoulder portion 14 to provide a nesting support for a patient's neck.

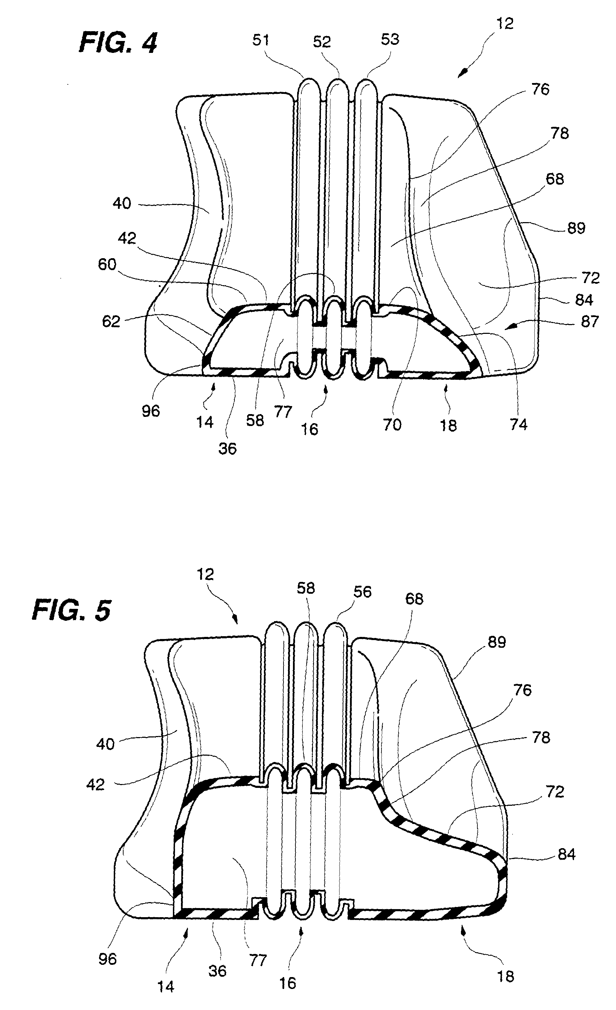

Most cervical injuries to patients involve the loss of the natural cervical curve forming a so called military neck or straight neck syndrome. This creates stress on the upper thoracic muscles, as these muscles are forced to hold the head upright. When the natural curve is in place, the head weight is distributed throughout the skeletal system. The body 12 of the cervical traction/stretch device 10 is constructed so that the patient's cervical curve is supported to relieve upper thoracic muscles from unnatural stress. The manner in which the shoulder portion 14 achieves this function at the center of the body 12 of the cervical traction/stretch device 10 is shown in FIG. 4 where the contoured surface 40 has a downwardly inclined portion 60 going up to a flat or slightly upwardly inclined surface portion 62 of the U-shaped surface 42.

Like the shoulder portion 14, the head portion 18 is also integrally formed with the bellows portion 16. The head portion 18 has a generally arcuate or semi-cylindrical U-shaped surface 68 having a portion 70 (see FIG. 3) that inclines slightly downwardly at the center to fit the cervical curve of the patient's neck and has a head receiving surface 72 having a center portion 74 that curves downwardly for mating with the cervical curve.

The U-shaped surface 68 extends toward the outer end of the head portion 18 a distance approximately 3/4 of an inch to one inch and forms a shoulder 76 on opposite sides of the U-shaped curved surface 68 but not at the center of the U-shaped curved surface 68. This can be best seen in FIGS. 4, 5 and 6.

As shown in FIG. 4, there is very little of the shoulder 76, with the U-shaped surface 68, 70, at the bottom of the U connecting with the surface portion 74 of the head receiving surface 72.

Also, as shown in FIGS. 4, 5 and 6, the inside of the inflatable cervical traction/stretch device 10 is a hollow chamber 77 which encompasses the combined interior of the shoulder portion 14, the bellows portion 16 and the head portion 18.

The thickness of the rubber material that forms the device 10 is generally uniform throughout the horizontal and vertical cross section of the shoulder portion 14 as shown in FIGS. 4, 5, 6 and 7. Also, the thickness of the material throughout the horizontal and vertical cross section of the head portion 18 is uniform. Finally, the thickness of the material throughout the horizontal and vertical cross section of the bellows portion 16 is also uniform. However, preferably, the rubber material is thicker in the shoulder portion 14 and the head portion 18 than in the bellows portion 16.

The thicker material in the shoulder portion 14 and the head portion 18 results in the bellows portion 16 being more readily expandable than the shoulder portion 14 and the head portion 18. Consequently, once the device 10 is inflated from the un-inflated condition to the first inflated condition to take the shape shown in FIGS. 2-7, further inflation of the device causes the bellows portion 16 to expand a greater amount than the head and shoulder portions 14 and 18. The shoulder portion 14 and the head portion 16 remain inflated in a shape similar to the first inflated condition and do not become distorted while further expanding.

The further expansion of the bellows portion 16 occurs through the expansion of the undulations in the bellows portion 16, e.g. undulations 51, 52 and 53, in a controlled manner, to cause even pressure against and separation of the shoulder portion 14 and the head portion 18. This expansion causes comfortable and therapeutic stretching to a patient's neck, while allowing the shoulder portion 14 and the head portion 18 to maintain the traction, stretching or lift surfaces created when the device 10 is inflated to the first condition.

As shown in FIG. 5, part way up either side of the U-shaped surface 68 the shoulder 76 is pronounced and is located at the junction between the U-shaped surface 68 and the specially contoured head receiving surface 72. The shoulder 76 at this location is adapted to bear against the occipital bone and defines in the head receiving surface an occipital cervical pressure or lift surface 78 just outwardly of the shoulder 76.

This shoulder 76 and the adjacent pressure or lift surface 78 on the head receiving surface 72 enables the head portion 18 to apply pressure at the region of the occipital bone of a patient on each side of the neck. It is believed that this pressure on the occipital bone applied with the cervical traction/stretch device 10 of the present invention also can alleviate or relieve headache pain.

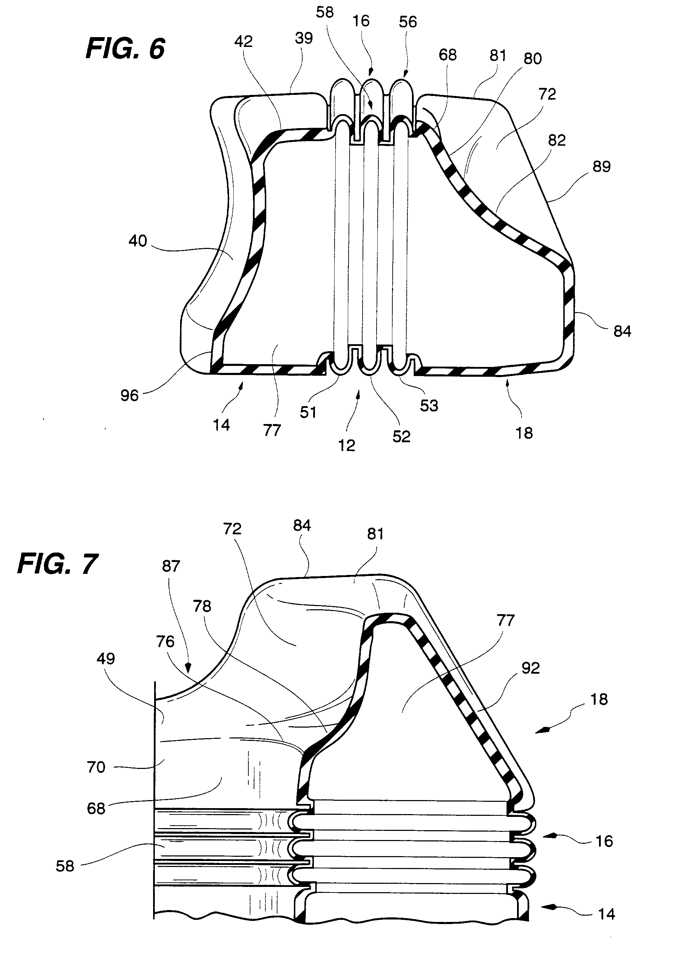

Looking now at FIGS. 4, 5 and 6 it will be appreciated that the body 12 has in the U-shaped openings and at the center of the U-shaped surfaces 42, 58, 68 the curved straight or inclined surface portions 62, 60, 42, 58, 70, and 74 for receiving the cervical curve of the neck (FIG. 4). Then as one moves to the left side or the right side of the U-shaped openings in the area of the U-shaped opening in the head portion 18, the head receiving surface 72 has the pronounced shoulder 76, the occipital bone receiving surface 78 and then a gentle sloping curving surface portion 80 (FIG. 6) for supporting the head above the occipital bone on each side of the head.

As shown in FIG. 6, near a top side 81 of the head portion 18, the U-shaped surface 68 slopes in a longitudinal direction downwardly and merges in or with a smooth downwardly extending curved surface portion 82 of the head receiving surface 72 which extends to an outer end 84 of the head portion 18 on each side of the head receiving surface 72.

The U-shaped opening 58 in the bellows portion 16 has, in the extended or inflated condition, a depth of between 3 and 4 inches and preferably 3 and 1/2 inches.

Looking now at FIG. 2, it will be apparent that the specially contoured head receiving surface 72 extends downwardly to a bottom 86 (FIG. 3) in the central area of the head portion 18 such that the head portion has a U-shaped notch 87 at the bottom thereof where the head of a patient can then rest on a flat surface.

Then the ends 84 of the head portion 18, on either side of the center thereof, has left and right end wall portions 88 extending upwardly from the flat bottom 86 of the head portion 18 to sloping end wall portions 89, 90 which slope upwardly and inwardly toward the top side 81 of the head portion 18.

Also, as best shown in FIG. 2 left and right outer sides 91, 92 of the head portion 18 are inclined from the bellows portion 16 inwardly and outwardly to the upwardly and inwardly inclined end wall portions 89, 90. On each of these left and right outer sides 91, 92 there is provided the patches 31, 32 (FIG. 3) of hook and loop attaching material of the type sold under the trademark VELCRO®. Each patch 31, 32 is located on the inclined side 91 or 92 adjacent the bottom 86.

If desired, the bottom 86 of the head portion 18 can have an inclined slot or flute 94 or 95 therein on either side of the U-shaped notch 87 as shown in FIG. 3.

As best shown in FIGS. 1, 8 and 9 the head strap 34 has a fabric attachment structure 36 of the type sold under the trademark VELCRO® on the inside 36 of the strap 34 which, at each end of the strap 34, is adapted to be received over a patient's forehead and secured to the patches 31, 32 to securely hold the patient's head to the head portion 18 of the body 12 of the cervical traction/stretch device 10 to achieve the greatest stretch or traction.

In the use of the cervical traction/stretch device 10 a user or patient will inflate the device from the un-inflated condition to the first inflated condition. Then the person will rotate the thumbscrew 28 to prevent air from escaping from the device 10. Then the person will place the inflated device 10 on a flat surface such as a floor or table and lay down on the floor or table with the cervical curve of the patient's neck received over the center of the U-shaped notch 87 formed by the U-shaped surfaces, i.e., over surfaces or surface portions 62, 60, 42, 58, 72, 74, in the shoulder portion 14, the bellows portion 16 and the head portion 18. Then the user or patient or a doctor or other medical technician will place the strap 34 over the head of the user or patient and secure it firmly to the patches 31, 32. Next, the user, or a medical technician will pump the hand-held bulb type air pump 22 to further inflate the device 10 to the second inflated condition where the bellows portion 16 is expanded to create traction on the cervical area of the patient's neck supported by the surface portions 62, 60, 42, 58, 72, 74, in the shoulder portion 14, the bellows portion 16 and the head portion 18.

If desired an electrically operated air pump (not shown) can be connected to the tubing 20 in place of the bulb type hand-operated air pump 22. Such an electronic pump will include a timer for cycling the electrical pump through intermittent pump and relief cycles thereby to apply intermittent traction/stretch to the patient's neck for treating soft tissue or disk dysfunctions, not limited to arthritis, of a patient. Intermittent traction/stretch is preferred by healthcare professionals as a method of treatment. In this modification, air can be pumped into or released out of the bellows portion 54 under the control of a timer having several different time cycles.

A user, patient or clinician can achieve some of the same benefits of electrically controlled intermittent traction/stretch by intermittently pumping the inflation bulb 22 and operating the release valve 26.

From the foregoing description, it will be apparent that the cervical traction/stretch device 10 of the present invention has a number of advantages, some of which have been described above and others of which are inherent in the invention.

Also from the foregoing description it will be apparent that modifications can be made to the cervical traction/stretch device 10 without departing from the teachings of the invention.

Accordingly the scope of the invention is only to be limited as necessitated by the accompanying claims.

## Claims

1. A cervical traction or stretch device comprising:
a hollow inflatable body made of an elastically expandable material;
said body having a shoulder engaging portion (14) and a head engaging portion (18);
a pumping mechanism connected to said body for pumping air into said body and for releasing air from said bellows;
characterized by:
a bellows portion (16) between said shoulder engaging portion (14) and said head engaging portion (18);
said bellows (16) having an undulated or pleated wall (51, 52, 53) with the undulations or pleats extending transversely of a user's neck; and
said bellows (16) having an inflated condition where said bellows (16) is expanded so that said shoulder engaging portion (14) and said head engaging portion (18) engage and act against and between a user's shoulder and head to extend the distance between said shoulder engaging portion (14) and said head engaging portion (18).

2. The cervical traction or stretch device of claim 1 characterized in that said pumping means (22) for pumping air into said bellows includes (16) a conduit (20) and a bulb air pump (22) at the outer end of said conduit; said bulb type air pump having a one way inlet valve and, at an inner end thereof, a manually operated relief valve (26).

3. The cervical traction or stretch device of claim 1 characterized in that said means (22) for pumping air into said bellows (16) includes means for intermittently pumping air into and releasing air out of said bellows (16) for treating soft tissue or disk dysfunctions, including arthritis, in the neck of the user.

4. The cervical traction or stretch device of claim 3 characterized in that said means (22) for pumping air into said bellows (16) includes an electrically operated pump and a timer for controller operation of said electrically operated pump whereby air can be intermittently pumped into and out of said bellows (169 for treating soft tissue or disk dysfunctions, including arthritis, in the neck of the a user.

5. The cervical traction or stretch device of claim 1 characterized in that said shoulder engaging portion (14) is defined by a shoulder portion at a lower side of said bellows (16) and said head engaging portion (18) is defined by a head portion (18) at an upper side of said bellows (16);
and said bellows (16) said head portion (18) and said shoulder portion (14) having aligned U-shaped openings (68, 58, 42).

6. The cervical traction or stretch device of claim 5 characterized in that said elastically expandable material is thicker (Fig. 5) in said shoulder portion (14) and in said head portion (18) than in said bellows (16) so that then said body (12) is inflated from a first inflated condition to a second inflated condition said bellows (16) expands more than said head and shoulder portions.

7. The cervical traction or stretch device of claim 5 characterized in that said shoulder portion (14) has an outer end surface (40, 49, 50) specially contoured to seat over the top center and front chest portions of the left and right shoulders of a user and bear against said left and right shoulders when said body (12) is inflated in a first inflated condition or a second inflated condition.

8. The cervical traction or stretch device of claim 5 characterized in that said aligned U-shaped openings (68, 58, 42) are defined by U-shaped surfaces and said U-shaped surfaces in the middle, bight area, of said U-shaped openings conform generally to the natural cervical curve of a user's neck receivable therein.

9. The cervical traction or stretch device of claim 5 characterized in that said head portion (18) has surface means (70, 72, 74, 76, 78) for receiving the neck and head of a user of the device, said means including contoured curved head receiving surfaces for receiving and supporting a user's or patient's head and support structure in said contoured surfaces including a shoulder (76) and adjacent lift surface (78) for engaging and exerting pressure against the occipital bone of a patient's head received in said head portion.

10. The cervical traction or stretch device of claim 5 characterized by further including a head strap (34), fastening means (31, 32) on each side of said head portion (18) and attachment means (36)on one side of said strap (34) adjacent at least each end of said strap for coupling with said fastening means (31, 32) on each side of said head portion.

## Patentansprüche

1. Zug- oder Dehnvorrichtung für die Halswirbelsäule bestehend aus: einem hohlen, aufblasbaren Körper aus einem elastischen, entfaltbaren Material;
wobei der Körper einen an der Schulter ansetzenden Teil (14) und einen am Kopf ansetzenden Teil (18) aufweist;
einem mit diesem Körper verbundenen Pumpmechanismus zum Pumpen von Luft in diesen Körper hinein und zum Auslassen von Luft aus dem Balg;
**dadurch gekennzeichnet,**
**dass** ein Balgteil (16) zwischen dem an der Schulter ansetzenden Teil (14) und dem am Kopf ansetzenden Teil (18) vorgesehen ist; dass der Balg (16) eine wellen- oder faltenförmige Wand (51, 52, 53) aufweist, wobei die Wellen oder Falten quer zum Hals des Anwenders verlaufen; und
dass der Balg (16) in einem aufgeblasenen Zustand derart entfaltet ist, dass der an der Schulter ansetzende Teil (14) und der am Kopf ansetzende Teil (18) angesetzt werden und gegen und zwischen Schulter und Kopf des Anwenders wirken, um somit den Abstand zwischen dem an der Schulter ansetzenden Teil (14) und dem am Kopf ansetzenden Teil (18) zu vergrößern.

2. Zug- oder Dehnvorrichtung für die Halswirbelsäule nach Anspruch 1, dadurch gekennzeichnet, dass die Pumpmittel (22) zum Pumpen von Luft in den Balg (16) eine Leitung (20) und einen Handblasebalg (22) am äußeren Ende der Leitung umfassen, wobei diese Luftpumpe mit Handblasebalg ein Rückschlageinlaßventil und an einer seiner Innenseiten ein handbetriebenes Entlastungsventil (26) besitzt.

3. Zug- oder Dehnvorrichtung für die Halswirbelsäule nach Anspruch 1, dadurch gekennzeichnet, dass die Mittel (22) zum Pumpen von Luft in den Balg (16) Mittel zum intermittierenden Pumpen von Luft in den Balg hinein und aus dem Balg (16) heraus umfassen, wobei diese Mittel zur Behandlung von Funktionsstörungen an Weichteilen oder Bandscheiben, einschließlich Arthritis, im Hals des Anwenders vorgesehen sind.

4. Zug- oder Dehnvorrichtung für die Halswirbelsäule nach Anspruch 3, dadurch gekennzeichnet, dass die Mittel (22) zum Pumpen von Luft in den Balg (16) eine elektrisch betriebene Pumpe und einen Zeitschalter für den gesteuerten Betrieb der elektrisch betriebenen Pumpe umfassen, wobei zur Behandlung von Funktionsstörungen an Weichteilen oder Bandscheiben, einschließlich Arthritis, im Hals des Anwenders Luft abwechselnd in den Balg hinein und aus dem Balg (16) heraus gepumpt werden kann.

5. Zug- oder Dehnvorrichtung für die Halswirbelsäule nach Anspruch 1, dadurch gekennzeichnet, dass der an der Schulter ansetzende Teil (14) durch einen an einer unteren Seite des Balges (16) vorgesehenen Schulterteil gebildet ist und dass der am Kopf ansetzende Teil (18) durch einen an einer oberen Seite des Balges (16) vorgesehenen Kopfteil (18) gebildet ist,
und dass der Balg (16), das Kopfteil (18) und das Schulterteil (14) fluchtende, U-förmige Öffnungen (68, 58, 42) aufweisen.

6. Zug- oder Dehnvorrichtung für die Halswirbelsäule nach Anspruch 5, dadurch gekennzeichnet, dass das elastisch entfaltbare Material in dem Schulterteil (14) und in dem Kopfteil (18) dicker (Fig. 5) ist als in dem Balg (16), so dass, wenn der Körper (12) von einem ersten aufgeblasenen Zustand in einen zweiten aufgeblasenen Zustand aufgeblasen wird, der Balg (16) sich mehr entfaltet als die Kopf- und Schulterteile.

7. Zug- oder Dehnvorrichtung für die Halswirbelsäule nach Anspruch 5, dadurch gekennzeichnet, dass der Schulterteil (14) eine äußere Endfläche (40, 49, 50) aufweist, die derart konturiert ist, dass sie auf der oberen Mitte und den vorderen Brustbereichen der linken und rechten Schulter eines Anwenders aufsitzt und an der linken und rechten Schulter in Anlage kommt, wenn der Körper (12) in einen ersten oder in einen zweiten aufgeblasenen Zustand aufgeblasen wird.

8. Zug- oder Dehnvorrichtung für die Halswirbelsäule nach Anspruch 5, dadurch gekennzeichnet, dass die fluchtenden U-förmigen Öffnungen (68, 58, 42) durch U-förmige Flächen ausgebildet sind, und dass die U-förmigen Flächen im mittleren, buchtförmigen Bereich der U-förmigen Öffnungen im Allgemeinen der natürlichen Halswirbelsäulenkrümmung des darin aufnehmbaren Halses eines Anwenders angepaßt sind.

9. Zug- oder Dehnvorrichtung für die Halswirbelsäule nach Anspruch 5, dadurch gekennzeichnet, dass der Kopfteil (18) Flächenmittel (70, 72, 74, 76, 78) zur Aufnahme von Hals und Kopf eines Anwenders der Vorrichtung aufweist, wobei diese Mittel gebogene Aufnahmeflächen für den Kopf zur Aufnahme und Stütze des Kopfes eines Anwenders oder Patienten und eine in diesen konturierten Flächen angeordnete Stützstruktur aufweisen, die eine Schulterfläche (76) und eine angrenzende Hebefläche (78) zum Ansetzen an und Druck ausüben auf den Hinterhauptsknochen des in diesem Kopfteil aufgenommenen Kopf des Patienten umfasst.

10. Zug- oder Dehnvorrichtung für die Halswirbelsäule nach Anspruch 5, dadurch gekennzeichnet, dass weiterhin ein Kopfbügel (34), Feststellmittel (31, 32) an jeder Seite des Kopfteils (18) und Befestigungsmittel (36) an einer Seite des Kopfbügels (34) angrenzend an mindestens jedem Ende des Bügels zur Verbindung mit den Befestigungsmitteln (31, 32) auf jeder Seite des Kopfteils vorgesehen sind.

## Revendications

1. Dispositif de traction ou d'extension cervicale comprenant:
un corps creux gonflable en un matériel élastique expansible ;
ledit corps ayant une partie se mettant en prise avec l'épaule (14) et une partie se mettant en prise avec la tête (18);
un mécanisme de pompage relié audit corps pour pomper de l'air dans ledit corps et pour faire s'échapper l'air dudit soufflet;
**caractérisé en ce que**
une partie de soufflet (16) est disposée entre la partie se mettant en prise avec l'épaule (14) et la partie se mettant en prise avec la tête (18);
le soufflet (16) ayant une paroi ondulée ou à plis (51, 52, 53), les ondes ou les plis s'étendant transversalement par rapport au cou de l'utilisateur; et
le soufflet (16) ayant un état de gonflement dans lequel le soufflet entre en expansion de sorte que la partie se mettant en prise avec l'épaule (14) et la partie se mettant en prise avec la tête (18) s'engagent entre et agissent contre l'épaule et la tête d'un utilisateur afin d'agrandir l'écart entre la partie se mettant en prise avec l'épaule (14) et la partie se mettant en prise avec la tête (18).

2. Dispositif de traction ou d'extension cervicale selon
la revendication 1 caractérisé en ce que les moyens de pompage (22) destinés à pomper de l'air dans le soufflet (16) comprennent une conduite (20) et une poire de gonflage (22) à l'extrémité extérieure de la conduite; cette pompe à air avec poire de gonflage ayant une valve d'admission unidirectionnelle et, à une extrémité intérieure, une valve d'échappement (26) actionnée à la main.

3. Dispositif de traction ou d'extension cervicale selon
la revendication 1 caractérisé en ce que les moyens (22) pour pomper de l'air dans le soufflet (16) comprennent des moyens pour pomper de l'air dans et faire s'échapper de l'air du soufflet (16) par intermittence et sont destinés à être employés dans le traitement des troubles fonctionnels des parties molles ou des disques intervertébraux, y compris l'arthrite, dans le cou de l'utilisateur.

4. Dispositif de traction ou d'extension cervicale selon
la revendication 3 caractérisé en ce que les moyens (22) pour pomper de l'air dans le soufflet (16) comprennent une pompe actionnée électriquement et un interrupteur horaire pour un régime contrôlé de la pompe actionnée électriquement, l'air pouvant être pompé par intermittence dans et hors du soufflet (16) pour le traitement des troubles fonctionnels de parties molles ou de disques intervertébraux, y compris l'arthrite, dans le cou d'un utilisateur.

5. Dispositif de traction ou d'extension cervicale selon
la revendication 1 caractérisé en ce que la partie se mettant en prise avec l'épaule (14) est définie par une partie épaule située au bas du soufflet (16) et que la partie se mettant en prise avec la tête (18) est définie par une partie tête (18) située en haut du soufflet (16); ledit soufflet (16), ladite partie tête (18) et ladite partie épaule (14) ayant des ouvertures (68, 58, 42) en forme de U disposées en alignement.

6. Dispositif de traction ou d'extension cervicale selon
la revendication 5 caractérisé en ce que le matériel élastique expansible est plus épais (Fig. 5) dans la partie épaule (14) et dans la partie tête (18) que dans ledit soufflet (16) de sorte que, lorsque le corps (12) est gonflé d'un premier état de gonflement vers un second état de gonflement, le soufflet (16) entre plus en expansion que les parties épaule et tête.

7. Dispositif de traction ou d'extension cervicale selon
la revendication 5 caractérisé en ce que ladite partie épaule (14) a une surface terminale extérieure (40, 49, 50) spécialement contournée pour reposer sur le centre supérieur et les parties ventrales des épaules gauche et droite d'un utilisateur et pour reposer contre lesdites épaules gauche et droite quand le corps (12) est gonflé pour lui faire atteindre un premier état de gonflement ou un second état de gonflement.

8. Dispositif de traction ou d'extension cervicale selon
la revendication 5 caractérisé en ce que lesdites ouvertures en forme de U disposées en alignement (68, 58, 42) sont définies par des surfaces en forme de U, lesdites surfaces en forme de U des ouvertures en forme de U situées dans la zone centrale formant une baie épousant généralement la courbure cervicale naturelle du cou de l'utilisateur qu'elles sont en mesure de recevoir.

9. Dispositif de traction ou d'extension cervicale selon
la revendication 5 caractérisé en ce que ladite partie tête (18) a des éléments de surface (70, 72, 74, 76, 78) pour recevoir le cou et la tête de l'utilisateur, ces éléments comprenant des surfaces de réception contournées pour la tête destinées à recevoir et soutenir la tête d'un utilisateur ou d'un patient, la structure de soutien dans lesdites surfaces contournées comprenant une surface épaule (76) et une surface d'élévation contiguë (78) destinée à entrer en prise avec et à exercer une pression sur l'os occipital de la tête d'un patient reçue dans ladite partie tête.

10. Dispositif de traction ou d'extension cervicale selon
la revendication 5 caractérisé en ce qu'il comprend de plus une bride pour la tête (34), des moyens de fixation (31, 32) de chaque côté de ladite partie tête (18) et des moyens d'attache (36) sur un côté de la bride (34) contigu à au moins chaque extrémité de la bride pour la liaison avec les moyens de fixation (31, 32) de chaque côté de ladite partie tête.
